# EUROPÄISCHE PATENTSCHRIFT

(11) **EP 0 638 233 B1**
(45) Veröffentlichungstag und Bekanntmachung des Hinweises auf die Patenterteilung: **06.08.1997**
(21) Anmeldenummer: 94890090.7
(22) Anmeldetag: 25.05.1994
(51) Int. Cl.: A01M 23/04, A01M 23/16, A01M 25/00

(54) **Falle für Kleintiere**
Trap for small animals
Piège pour petits animaux

(30) Priorität: 11.08.1993 AT 1604/93
(43) Veröffentlichungstag der Anmeldung: 15.02.1995
(73) Patentinhaber: Prochazka, Werner, 1130 Wien (AT)
(72) Erfinder: Prochazka, Werner, 1130 Wien (AT)
(74) Vertreter: Kliment, Peter, Dipl.-Ing.

(56) Entgegenhaltungen:
- AT-A- 391 586
- US-A- 4 318 241
- US-A- 4 741 121

## Beschreibung

Die Erfindung betrifft eine Falle für Kleintiere, insbesondere Mäuse, mit einem nach oben offenen Gehäuse, das einen das Kleintier aufnehmenden Käfig aufweist, der eine durch eine schwenkbare Eingangsklappe verschließbaren Eingangsöffnung aufweist, mit einem zu öffnenden Deckel, der das Gehäuse abschließen kann, und mit einem Fangmechanismus zum Einbringen des Kleintiers in den Käfig, wobei eine Vergasungsvorrichtung im Gehäuse vorgesehen ist, die nach Verschluß der Eingangsöffnung den Käfig mit für das Kleintier tödlichem Gas füllt, und die eine vom Kleintier über ein Auslöseorgan betätigbare Einwurfeinrichtung aufweist, welche durch das Einwerfen einer Tablette aus einer in Reaktion mit einer Flüssigkeit gasbildenden Chemikalie in einen im Gehäuse vorgesehenen Flüssigkeitstank das tödliche Gas bildet.

Eine Falle für Kleintiere der eingangs genannten Art ist aus der AT-PS 391 586 bekannt. Das Prinzip der Vorrichtung beruht auf folgender Arbeitsweise: Sobald das Kleintier in die Eingangsöffnung eintritt, löst es über eine Wippe die Eingangsklappe aus, sodaß das Kleintier im Gehäuse gefangen ist. Über einen weiteren Auslösemechanismus betätigt das Kleintier eine Einwurfeinrichtung, welche eine Tablette in eine Flüssigkeit befördert, wobei eine Reaktion stattfindet, die ein für das Kleintier tödliches Gas erzeugt.

Derartige Fallen haben den Vorteil, daß auch ein köderloser Einsatz möglich ist, und daß bei einem gefangenen Tier ein schneller und schmerzfreier Tod eintritt. Die Tablette besteht dabei aus ungiftigen, gasbildenden Wirkstoffen, wodurch spielende Kinder in keiner Weise durch Schadstoffe gefährdet sind.

Allerdings hat die in der AT-PS 391 586 beschriebene Falle den Nachteil eines hohen Herstellungaufwandes und damit verbunden, relativ hoher Herstellungskosten. Zudem kommt, daß die Handhabung und Reinigung der Falle nicht gerade einfach ist.

Aufgabe der Erfindung ist es, diese Nachteile zu vermeiden und eine gegenüber dem Stand der Technik einfachere Falle zu konzipieren, wobei die genannten Vorteile weiterbestehen bleiben.

Erfindungsgemäß ist daher vorgesehen, daß die Einwurfeinrichtung eine schwenkbare Tragplatte aufweist, auf welche die Tablette auflegbar ist, daß die Tragplatte in dem Flüssigkeitstank über der Flüssigkeit angeordnet ist, und daß die Tragplatte nach oder gemeinsam mit der Eingangsklappe kippbar ist, um das Einwerfen der Tablette in die Flüssigkeit zu bewirken, wobei die Schwenkbewegung der Tragplatte direkt oder indirekt an die Schwenkbewegung der Eingangsklappe gekoppelt ist. Durch die sehr einfach aufgebaute Einwurfeinrichtung und die Koppelung der Bewegung der Tragplatte mit der Eingangsklappe, ist ein sehr kompakter und einfacher Aufbau der Falle und damit eine kostengünstige Herstellung möglich. Das Kleintier kann durch Betätigen eines einzigen Auslöseorganes sowohl die Schwenkbewegung der Tragplatte, als auch die Schwenkbewegung der Eingangsklappe einleiten. Vorzugsweise ist dabei vorgesehen, daß die Schwenkachse der Tragplatte von der Schwenkachse der Eingangsklappe direkt oder indirekt angetrieben ist, wobei in weiterer Ausbildung, die Schwenkachsen der Tragplatte und der Eingangsplatte in Form einer einzigen gemeinsamen Achse ausgebildet sein können. Die Tragplatte und die Eingangsplatte können dabei aus einem Stück geformt sein.

In einer bevorzugten Ausführungsvariante ist vorgesehen, daß die Falle höchstens aus den vier folgenden Teilen besteht, nämlich dem Käfig und den Flüssigkeitstank beinhaltenden Gehäuse, dem Deckel, einer aus der Eingangsklappe, der Tragplatte und der diese miteinander verbindenden Achse bestehenden Einheit, sowie dem vom Kleintier zu betätigenden Auslöseorgan zur Schließung der Eingangsklappe, wobei die Schlieβung ausschließlich durch das Eigengewicht der vorigen Einheit zusammen mit dem Gewicht der Tablette erfolgt. Durch die geringe Anzahl der Teile wird die Wartung und Reinigung sowie die Herstellung und der Zusammenbau der Falle sehr vereinfacht.

Eine weitere Vereinfachung wird erreicht, wenn der Deckel und das Gehäuse einstückig ausgebildet sind, wobei beide durch ein Scharnier beweglich verbunden sind.

Um eine rasche Wirkung des tödlichen Gases zu erreichen, sollte das Gehäuse durch den Deckel abdichtend verschließbar sein.

In einer bevorzugten Ausführungsvariante ist vorgeshen, daß der Flüssigkeitstank und der Käfig mittels einer Zwischenwand des Gehäuses getrennt sind und daß eine im Deckel und/oder im Gehäuse angeordnete Verbindungsöffnung oder -leitung vorgesehen ist, und daß das Volumen des Flüssigkeitstanks kleiner ist als das Volumen des Käfigs, so daß das erzeugte Gas fast vollständig von dem Flüssigkeitstank in den Käfig fließen kann. Das Gehäuse der Falle schließt somit nur die beiden Räume, Käfig und Flüssigkeitstank ein. Eine komplizierte Unterteilung des Gehäuseinneren ist daher nicht erforderlich.

Eine sichere Funktion der Falle ist gewährleistet, wenn die Tablette als gasbildenden Wirkstoff ein Salz der Kohlensäure, vorzugsweise Natriumhydrogencarbonat, enthält und die Flüssigkeit im wesentlichen aus Wasser besteht, das vorzugsweise einen Zusatz von Zitronensäure-1-hydrat enthält. Als tödliches Gas wird so Kohlendioxid erzeugt, das vollkommen ungiftig ist und auch sonst bei der Verwendung keinerlei Probleme verursacht. Der Effekt von Kohlendioxid besteht darin, daß dieses Gas den für die Atmung erforderlichen Sauerstoff verdrängt, wodurch der Tod durch Ersticken eintritt. Dieser Tod ist jedoch keineswegs qualvoll, sondern eher von euphorischen Zuständen begleitet, wie sie etwa vom Höhenrauch bekannt sind. Außerdem sind die erforderlichen Chemikalien, wie Natriumhydrogencarbonat, einfach zu handhaben, ungefährlich und billig. Im Prinzip ist die Füllung des Tanks mit Wasser ausreichend. Zur Beschleunigung der Gasbildungsreaktion ist jedoch die Zugabe eines sauren Mediums, wie etwa Zitronensäure-1-hydrat vorteilhaft.

In einer sehr einfach herzustellenden Ausführungsvariante ist vorgesehen, daß das Gehäuse der Falle im wesentlichen so geformt ist, daß, in Eingangsrichtung, der Käfig und der Flüssigkeitstank nebeneinander angeordnet sind, wobei die Eingangsöffnung unmittelbar neben einer Hochkante des Gehäuses, vorzugsweise auf gleicher Front mit einer den Flüssigkeitstank zum Teil bildenden Seitenwand des Gehäuses, angeordnet ist, und daß die Falle vom Typ der köderlosen Fallen ist.

Es ist eine Erfahrungstatsache, daß Mäuse und verwandte Kleintiere Räume nicht durchqueren, sondern soweit als möglich die Wände entlanglaufen. Aus diesem Grunde ist die Eingangsöffnung so angeordnet, daß sie bei richtig aufgestellter Falle unmittelbar neben der Wand des Raumes liegt. Die Maus wird also zwangsläufig und ohne von einem Köder gelockt worden zu sein, in die Falle hineinlaufen.

In einer sehr wirkungsvollen Ausführungsvariante ist vorgesehen, daß das Auslöseorgan eine unmittelbar hinter der Eingangsöffnung angebrachte Wippe aufweist, die in ihrer Ruhestellung die Eingangsklappe, vorzugsweise über eine Haltelasche, in der geöffneten Position hält und die unter Belastung etwa durch ein Kleintier in eine andere Stellung kippt und die Eingangsklappe freigibt.

Sämtliche Teile der Falle können aus Kunststoff hergestellt sein. Dies hat den Vorteil einer sehr einfachen und kostengünstigen Herstellung und ermöglicht eine pflegeleichte Handhabung der Falle. Dies trägt wesentlich zur Hygiene bei.

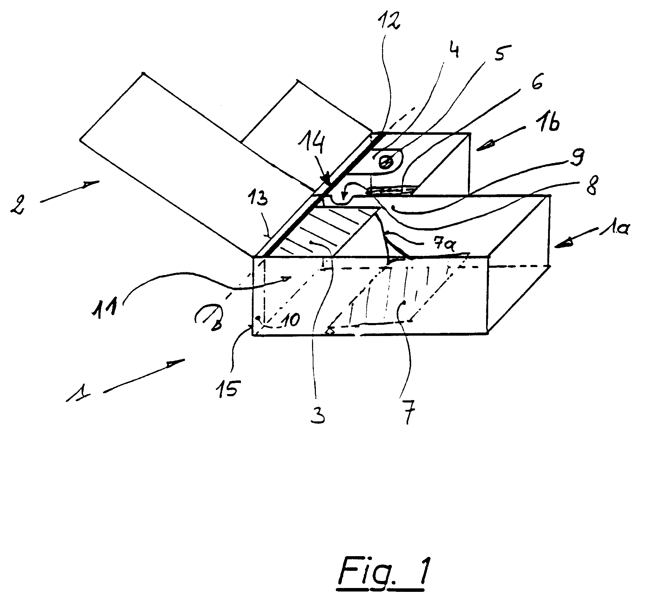

Im folgenden wird die Erfindung anhand eines in den Figuren dargestellten Ausführungsbeispieles näher erläutert. Es zeigen Fig. 1 eine erfindungsgemäße Falle schematisch in axionometrischer Darstellung, Fig. 2 einen Querschnitt durch eine erfindungsgemäße Falle im Aufriß nach der Linie II-II in Fig. 3, Fig. 3 eine Ansicht auf die Falle von oben bei abgenommenem Deckel.

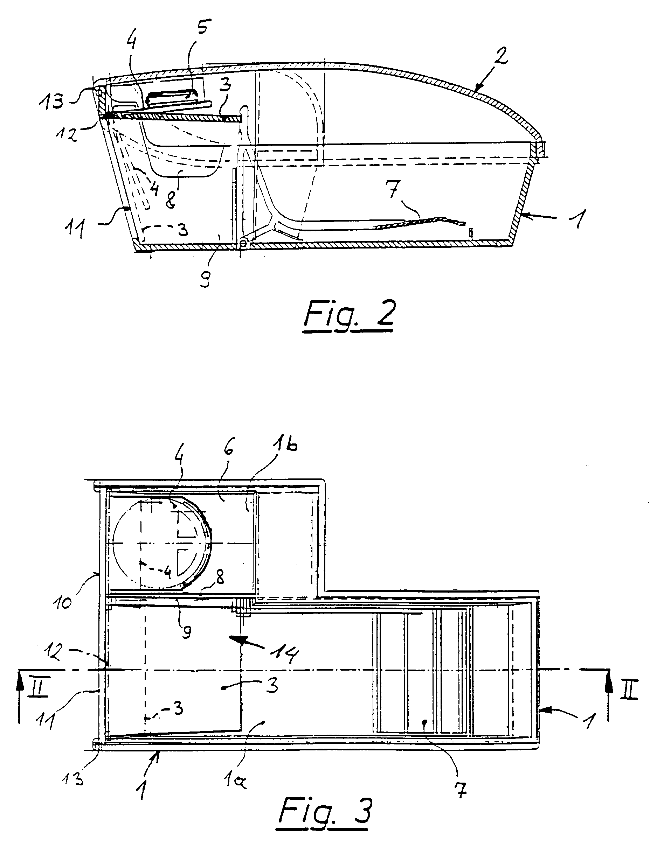

Die Falle besteht im wesentlichen aus dem Gehäuse 1, dem Deckel 2, der Eingangsklappe 3, welche über die Achse 12 mit der Tragplatte 4 verbunden ist, sowie dem aus der Wippe 7a und der Haltelasche 7b bestehenden Auslösorgan 7, durch welches das Kippen der Eingangsklappe 3 und der Tragplatte 4 ausgelöst wird. Die gekippte Lage der Eingangsklappe 3 und der Tragplatte 4 ist in den Fig. 2 und 3 strichliert eingezeichnet. Das Gehäuse 1 ist unterteilt in den Käfig 1a und den Flüssigkeitstank 1b, welche durch die Zwischenwand 9 bis auf eine Verbindungsöffnung 8 voneinander getrennt sind. Die Eingangsöffnung 11 ist nahe der Hochkante 15 in der Seitenwand 10 des Gehäuses vorgesehen.

Zur Vorbereitung der Falle wird der über das Scharnier 13 mit dem Gehäuse 1 verbundene Deckel 2 von dem Gehäuse 1 abgehoben, dann wird Flüssigkeit 6 in den Flüssigkeitstank 1b gefüllt und eine Tablette 5 auf die Tragplatte 4 gelegt. Die Eingangsklappe 3 wird mittels der Wippe 7a und der Haltelasche 7b in der offenen Stellung fixiert und das Gehäuse 1 durch den Deckel 2 wieder geschlossen.

Ein Kleintier tritt durch die Eingangsöffnung 11 in den Käfig 1a ein. Bei einer Belastung des hinteren Abschnittes der Wippe 7a durch das Kleintier gibt die Haltelasche 7b des Auslöseorganes 7 die Eingangsklappe 3 frei, die dann in ihre geschlossene Position fällt. Gleichzeitig wird über die Achse 12 die Tragplatte 4 mit der Eingangsklappe 3 mitgekippt, wodurch die auf der Tragplatte 4 positionierte Tablette 5 in die Flüssigkeit 6 des Flüssigkeitstankes 1b fällt. Die Chemikalie der Tablette 5 reagiert dann mit der Flüssigkeit 6 und setzt das für das Kleintier tödliche Gas frei. Dieses Gas strömt über die Verbindungsöffnung 8 vom Flüssigkeitstank 1b in den Käfig 1a, wo das Tier eingeschlossen ist und schließlich getötet wird.

## Patentansprüche

1. Falle für Kleintiere, insbesondere Mäuse, mit einem nach oben offenen Gehäuse (1), das einen das Kleintier aufnehmenden Käfig (1a) aufweist, der eine durch eine schwenkbare Eingangsklappe (3) verschließbaren Eingangsöffnung (11) aufweist, mit einem zu öffnenden Deckel (2), der das Gehäuse (1) abschließen kann, und mit einem Fangmechanismus zum Einbringen des Kleintiers in den Käfig (1a), wobei eine Vergasungsvorrichtung im Gehäuse (1) vorgesehen ist, die nach Verschluß der Eingangsöffnung (11) den Käfig (1a) mit für das Kleintier tödlichem Gas füllt, und die eine vom Kleintier über ein Auslöseorgan (7) betätigbare Einwurfeinrichtung aufweist, welche durch das Einwerfen einer Tablette (5) aus einer in Reaktion mit einer Flüssigkeit (6) gasbildenden Chemikalie in einen im Gehäuse (1) vorgesehenen Flüssigkeitstank (1b) das tödliche Gas bildet, **dadurch gekennzeichnet**, daß die Einwurfeinrichtung eine schwenkbare Tragplatte (4) aufweist, auf welche die Tablette (5) auflegbar ist, daß die Tragplatte (4) in dem Flüssigkeitstank (1b) über der Flüssigkeit (6) angeordnet ist, und daß die Tragplatte (4) nach oder gemeinsam mit der Eingangsklappe (3) kippbar ist, um das Einwerfen der Tablette (5) in die Flüssigkeit (6) zu bewirken, wobei die Schwenkbewegung der Tragplatte (4) direkt oder indirekt an die Schwenkbewegung der Eingangsklappe (3) gekoppelt ist.

2. Falle nach Anspruch 1, **dadurch gekennzeichnet,** daß die Schwenkachse der Tragplatte (4) von der Schwenkachse der Eingangsklappe (3) direkt oder indirekt angetrieben ist.

3. Falle nach Anspruch 2, **dadurch gekennzeichnet**, daß die Schwenkachsen der Tragplatte (4) und der Eingangsplatte (3) in Form einer einzigen gemeinsamen Achse (12) ausgebildet sind.

4. Falle nach Anspruch 3, **dadurch gekennzeichnet,** daß die Falle höchstens aus den vier folgenden Teilen besteht, nämlich dem den Käfig (1a) und den Flüssigkeitstank (1b) beinhaltenden Gehäuse (1), dem Deckel (2), einer aus der Eingangsklappe (3), der Tragplatte (4) und der diese miteinander verbindenden Achse (12) bestehenden Einheit (14), sowie dem vom Kleintier zu betätigenden Auslöseorgan (7) zur Schließung der Eingangsklappe (3), wobei die Schließung ausschließlich durch das Eigengewicht der vorigen Einheit (14) zusammen mit dem Gewicht der Tablette (5) erfolgt.

5. Falle nach Anspruch 4, **dadurch gekennzeichnet**, daß der Deckel (2) und das Gehäuse (1) einstückig ausgebildet sind, wobei beide durch ein Scharnier (13) beweglich verbunden sind.

6. Falle nach einem der Ansprüche 1 bis 5, **dadurch gekennzeichnet,** daß das Gehäuse (1) durch den Deckel (2) abdichtend verschließbar ist.

7. Falle nach Anspruch 5 oder 6, **dadurch gekennzeichnet,** daß der Flüssigkeitstank (1b) und der Käfig (1a) mittels einer Zwischenwand (9) des Gehäuses (1) getrennt sind und daß eine im Deckel (2) und/oder im Gehäuse (1) angeordnete Verbindungsöffnung oder -leitung (8) vorgesehen ist, und daß das Volumen des Flüssigkeitstanks (1b) kleiner ist als das Volumen des Käfigs (1a), so daß das erzeugte Gas fast vollständig von dem Flüssigkeitstank (1b) in den Käfig (1a) fließen kann.

8. Falle nach einem der Ansprüche 1 bis 7, **dadurch gekennzeichnet,** daß die Tablette (5) als gasbildenden Wirkstoff ein Salz der Kohlensäure, vorzugsweise Natriumhydrogencarbonat, enthält und daß die Flüssigkeit (6) im wesentlichen aus Wasser besteht, das vorzugsweise einen Zusatz von Zitronensäure-1-hydrat enthält.

9. Falle nach einem der Ansprüche 1 bis 8, **dadurch gekennzeichnet,** daß das Gehäuse (1) der Falle im wesentlichen so geformt ist, daß, in Eingangsrichtung, der Käfig (1a) und der Flüssigkeitstank (1b) nebeneinander angeordnet sind, wobei die Eingangsöffnung (11) unmittelbar neben einer Hochkante (15) des Gehäuses (1), vorzugsweise auf gleicher Front mit einer den Flüssigkeitstank (1b) zum Teil bildenden Seitenwand (10) des Gehäuses (1), angeordnet ist, und daß die Falle vom Typ der köderlosen Fallen ist.

10. Falle nach Anspruch 5 oder 6, **dadurch gekennzeichnet,** daß das Auslöseorgan (7) eine unmittelbar hinter der Eingangsöffnung (11) angebrachte Wippe (7a) aufweist, die in ihrer Ruhestellung die Eingangsklappe (2), vorzugsweise über eine Haltelasche (7b), in der geöffneten Position hält und die unter Belastung etwa durch ein Kleintier in eine andere Stellung kippt und die Eingangsklappe (3) freigibt.

11. Falle nach einem der Ansprüche 1 bis 10, **dadurch gekennzeichnet,** daß sämtliche Teile der Falle aus Kunststoff hergestellt sind.

## Claims

1. A trap for small animals, in particular mice, with a casing (1) which is open at the top and comprises a cage (1a) for receiving the small animal, which cage is provided with an entrance opening (11) which is closable by a swivellable entrance flap (3), with a lid (2) to be opened which can close the casing (1) and with a catching mechanism for introducing the small animal into the cage (1a), with a gassing apparatus being provided in the casing (1) which after the closure of the entrance opening (11) fills the cage (1a) with a gas which is deadly for the small animal and which is provided with a throw-in device actuatable by the small animal by way of a release member (7) which by throwing in a tablet (5) forms the deadly gas by a chemical forming a gas in reaction with a liquid (6) in a liquid tank (1b) provided in the casing (1), characterized in that the throw-in device is provided with a swivellable carrier plate (4) on which the tablet (5) can be placed, that the carrier plate (4) is arranged in the liquid tank (1b) above the liquid (6) and that the carrier plate (4) is tiltable after or jointly with the entrance flap (3) in order to effect the throwing in of the tablet (5) into the liquid, with the swivelling movement of the carrier plate (4) being coupled directly or indirectly to the swivelling movement of the entrance flap (3).

2. A trap as claimed in claim 1, characterized in that the swivelling axle of the carrier plate (4) is driven directly or indirectly by the swivelling axle of the entrance flap (3).

3. A trap as claimed in claim 2, characterized in that the swivelling axles of the carrier plate (4) and the entrance plate (3) are arranged in the form of a single common axle (12).

4. A trap as claimed in claim 3, characterized in that the trap consists of a maximum of the four following parts, namely the casing (1) comprising the cage (1a) and the liquid tank (1b), the lid (2), and a unit (14) consisting of the entrance flap (3), the carrier plate (4) and the axle (12) jointly connecting the same, and the release member (7) to be actuated by the small animal for closing the entrance flap (3), with the closure occurring exclusively by the own weight of the aforementioned unit (14) in combination with the weight of the tablet (5).

5. A trap as claimed in claim 4, characterized in that the lid (2) and the casing (1) are made from one piece, with both being movably connected to one another by a hinge (13).

6. A trap as claimed in one of the claims 1 to 5, characterized in that the casing (1) is closeable in a sealed manner by the lid (2).

7. A trap as claimed in claim 5 or 6, characterized in that the liquid tank (1b) and the cage (1a) are separated by means of an intermediate wall (9) of the casing (1) and that there is provided a connecting opening or line (8) arranged in the lid (2) and/or in the casing (1) and that the volume of the liquid tank (1b) is smaller than the volume of the cage (1a), so that the gas produced can flow nearly completely from the liquid tank (1b) into the cage (1a).

8. A trap as claimed in one of the claims 1 to 7, characterized in that the tablet (5) comprises a salt of carbonic acid as the gas-forming agent, preferably sodium hydrogen-carbonate, and that the liquid (6) substantially consists of water which preferably contains an addition of citric acid-1-hydrate.

9. A trap as claimed in one of the claims 1 to 8, characterized in that the casing (1) of the trap is shaped substantially in such a way that, as seen in the entering direction, the cage (1a) and the liquid tank (1b) are arranged adjacent to one another, with the entrance opening (11) being arranged directly adjacent to an upright edge (15) of the casing (1), preferably on the same frontage with a side wall (10) of the casing (1) partly forming the liquid tank (1b), and that the trap is of the type of bait-free traps.

10. A trap as claimed in claim 5 or 6, characterized in that the release member (7) is provided with a rocker (7a) attached directly behind the entrance opening (11), which in its idle position holds the entrance flap (2), preferably by way of a holding bracket (7b), in the opened position and which under the load of a small animal, for example, tilts into another position and releases the entrance flap (3).

11. A trap as claimed in one of the claims 1 to 10, characterized in that all parts of the trap are made from plastic material.

## Revendications

1. Piège pour petits animaux, notamment pour souris, avec un boîtier (1) ouvert vers le haut qui comporte une cage (1a) destinée à recevoir le petit animal et ayant un orifice d'entrée (11) refermable à l'aide d'un clapet d'entrée pivotant (3), avec un couvercle (2) ouvrant qui peut fermer le boîtier (1), et avec un mécanisme de capture destiné à introduire le petit animal dans la cage (1a), un dispositif de gazage étant prévu dans le boîtier (1), dispositif qui, après la fermeture de l'orifice d'entrée (11), remplit la cage (1a) d'un gaz mortel pour le petit animal, et qui présente un moyen d'introduction actionnable par le petit animal par l'intermédiaire d'un déclencheur (7) et produisant le gaz mortel par l'introduction, dans un réservoir (1b) prévu à l'intérieur du boîtier (1), d'un comprimé (5) qui consiste en un agent chimique produisant le gaz par réaction avec un liquide (6), **caractérisé en ce que** le moyen d'introduction comporte une plaque d'appui pivotante (4), sur laquelle le comprimé (5) est posé, en ce que la plaque d'appui (4) se trouve dans le réservoir (1b) au-dessus du liquide (6) et en ce que la plaque d'appui (4) peut être basculée après ou avec le clapet d'entrée (3) afin d'introduire le comprimé (5) dans le liquide (6), le mouvement pivotant de la plaque d'appui (4) etant lié directement ou indirectement au mouvement pivotant du clapet d'entrée (3).

2. Piège selon la revendication 1, **caractérisé en ce que** l'axe de pivotement de la plaque d'appui (4) est commandé directement ou indirectement par l'axe de pivotement du clapet d'entrée (3).

3. Piège selon la revendication 2, **caractérisé en ce que** les axes de pivotement de la plaque d'appui (4) et de la plaque d'entrée (3) sont construits sous forme d'un seul axe commun (12).

4. Piège selon la revendication 3, **caractérisé en ce que** le piège est composé au maximum des quatre parties suivantes, à savoir le boîtier (1) comportant la cage (1a) et le réservoir (1b), le couvercle (2), un bloc (14) comprenant le clapet d'entrée (3), la plaque d'appui (4) et l'axe (12) reliant ces deux derniers, ainsi que le déclencheur (7) à actionner par le petit animal et destiné à fermer le clapet d'entrée (3), la fermeture étant exclusivement réalisée par l'action du poids propre dudit bloc (14) en commun avec le poids du comprimé (5).

5. Piège selon la revendication 4, **caractérisé en ce que** le couvercle (2) et le boîtier (1) sont construits en une seule part, les deux étant reliés de façon mobile par une charnière (13).

6. Piège selon l'une des revendications 1 à 5, **caractérisé en ce que** le boîtier (1) peut être fermée de façon étanche par le couvercle (2).

7. Piège selon la revendication 5 ou 6, **caractérisé en ce que** le réservoir (1b) et la cage (1a) sont séparés à l'aide d'une cloison (9) du boîtier (1) et en ce qu'un orifice ou une conduite de liaison (8) agencé(e) dans le couvercle (2) et/ou le boîtier (1) est prévu(e) et en ce que le volume du réservoir (1b) est inférieur à celui de la cage (1a), de manière que la quasi-totalité du gaz qui se forme peut s'écouler du réservoir (1b) dans la cage (1a).

8. Piège selon l'une des revendications 1 à 7, **caractérisé en ce que** le comprimé (5) contient un sel de l'acide carbonique, de préférence du bicarbonate de sodium, comme substance produisant le gaz et en ce que le liquide (6) consiste essentiellement en eau qui, de préférence, contient de l'acide citrique monohydraté en tant qu'additif.

9. Piège selon l'une des revendications 1 à 8, **caractérisé en ce que** le boîtier (1) du piège est essentiellement conçu de façon que, dans la direction de l'entrée, la cage (1a) et le réservoir (1b) sont placés l'un à côté de l'autre, l'orifice d'entrée (11) étant placé directement à côté d'un bord vertical (15) du boîtier (1) et, de préférence, forme un seul front avec une face latérale (10) du boîtier (1) formant partiellement le réservoir (1b) et en ce que le piège est du type des pièges sans appât.

10. Piège selon la revendication 5 ou 6, **caractérisé en ce que** le déclencheur (7) est muni d'une bascule (7a) placée directement derrière l'orifice d'entrée (11) qui, dans sa position de repos, maintient le clapet d'entrée (2) en position ouverte, de préférence à l'aide d'une patte d'arrêt (7b), et qui, sous l'action d'une charge, par exemple, celle d'un petit animal, bascule dans une autre position en libérant le clapet d'entrée (3).

11. Piège selon l'une des revendications 1 à 10, **caractérisé en ce que** toutes les parties du piège sont réalisées en matière plastique.
